# EUROPÄISCHE PATENTSCHRIFT

(11) **EP 0 978 294 B1**
(45) Veröffentlichungstag und Bekanntmachung des Hinweises auf die Patenterteilung: **15.12.2004**
(21) Anmeldenummer: 99111026.3
(22) Anmeldetag: 11.06.1999
(51) Int. Cl.: A61N 5/10

(54) **Vorrichtung und Verfahren zum Steuern einer Bestrahlungseinrichtung**
Device and method for controlling an irradiation apparatus
Dispositif et procédé pour commander un appareil d'irradiation

(30) Priorität: 04.08.1998 DE 19835209
(43) Veröffentlichungstag der Anmeldung: 09.02.2000
(73) Patentinhaber: Gesellschaft für Schwerionenforschung mbH, 64291 Darmstadt (DE)
(72) Erfinder: Badura, Eugen, 64291 Darmstadt (DE); Brand, Holger, 64291 Darmstadt (DE); Essel, Hans-Georg, 64291 Darmstadt (DE); Haberer, Thomas, 64291 Darmstadt (DE); Hoffmann, Jan, 64291 Darmstadt (DE); Ott, Wolfgang, 64291 Darmstadt (DE); Poppensieker, Klaus, 64291 Darmstadt (DE)
(74) Vertreter: Boeters, Hans Dietrich, Dr.

(56) Entgegenhaltungen:
- EP-A- 0 143 993
- WO-A-96/25201
- DE-A- 19 633 744
- US-A- 4 477 882
- US-A- 5 017 789
- SHEA ET AL.: "Control system for the neutron therapy facility at fermilab" PROCEEDINGS OF THE 1989 IEEE PARTICLE ACCELERATOR CONFERENCE, Bd. 3, 20. März 1989 (1989-03-20), Seiten 1660-1662, XP002148375
- MATSU'URA J: "SYSTEMS FOR OVERALL CONTROL AND BEAM TRANSPORT OF THE HIMAC" MITSUBISHI ELECTRIC ADVANCE,JP,TOKYO, Bd. 72, September 1995 (1995-09), Seiten 5-7, XP000905408

## Beschreibung

Die vorliegende Erfindung betrifft eine Vorrichtung und ein Verfahren zum Steuern einer Bestrahlungseinrichtung.

Eine derartige Vorrichtung findet Anwendung insbesondere in einem System für Strahlentherapie zur Tumorbehandlung, wie beispielsweise aus US 5 017 789 bekannt. Als Strahlen kommen Teilchenstrahlen wie z.B. Protonen oder schwere Ionen infrage. In der Tumortherapie bieten Teilchenstrahlen gegenüber elektromagnetischen Strahlen besondere physikalische Vorteile; so haben sie ein sogenanntes "invertiertes" Dosisprofil, d.h. die im Zielvolumen - dem Tumorgewebe - deponierte Strahlendosis wächst mit zunehmender Eindringtiefe und hat kurz vor der maximalen Reichweite ein scharfes Maximum. Bei elektromagnetischen Strahlen hingegen nimmt die deponierte Strahlendosis mit wachsender Eindringtiefe ab, und ein großer Teil der Strahlendosis wird im gesunden Gewebe vor und hinter dem Tumor deponiert. Außerdem werden Teilchenstrahlen beim Durchgang durch dicke Gewebeschichten wesentlich weniger abgelenkt und können mit magnetischen Linsen extrem genau auf den Tumor gebündelt werden. Teilchenstrahlen können somit prinzipiell besser auf ein Zielvolumen fokussiert werden als elektromagnetische Strahlen. Die schweren Ionen, wie z.B. Kohlenstoff- oder Sauerstoff-Ionen, besitzen gegenüber den Protonen darüber hinaus noch bedeutende biologische Vorzüge in der Abtötung besonders strahlungsresistenter Tumorzellen.

Zur exakten Verteilung der Schwerionen-Strahlendosis über das Zielvolumen ist es bekannt, ähnlich wie bei der Tomographie nach der sogenannten Rasterscan-Methode vorzugehen. Eine Darstellung dieser Methode findet sich in einem Artikel von Th. Haberer, W. Becher, D. Schardt, D. Kraft mit dem Titel Magnetic scanning system for heavy ion therapy", erschienen in Nuclear Instruments and Methods in Physics Research A330 (1993) S. 296-305. Hierbei wird das Zielvolumen in einzelne Schichten gleicher Teilchenreichweite "zerlegt". In jeder Schicht wird ein Raster von Punkten definiert, für welche jeweils eine durch die Bestrahlung zu deponierende Teilchenzahl (Strahldosis) festgelegt wird. Die Gesamtheit der Punkte sowie die ihnen zugeordneten Teilchenzahlen und Teilchenenergien, d.h. die Verteilung der Teilchenzahlen über das gesamte Zielvolumen, bilden einen Bestrahlungsplan. Beginnend mit der hintersten Schicht tastet der Teilchenstrahl, von einem Paar magnetischer Ablenkungseinrichtungen abgelenkt, jede Schicht rasterartig ab. Hierbei wird die Dosis von Punkt zu Punkt in jeder Schicht dadurch variiert, daß der Teilchenstrahl so lange auf einen Punkt gerichtet wird, bis der Teilchenzahl-Sollwert gemäß dem Bestrahlungsplan erreicht ist.

Problematisch ist jedoch die Realisierung einer exakten, zuverlässigen und hinreichend schnellen Steuerung der Bestrahlungseinrichtung. Es sei angemerkt, daß im folgenden der Begriff des Steuerns einer Einrichtung für das Steuern und/oder das Kontrollieren derselben verwendet wird.

Auch war es bislang ein Problem, die Strahlablenkungsund Teilchenzahl-Steuereinrichtungen miteinander derart zu synchronisieren, daß sie während des Verlaufs der Bestrahlung jeweils zur selben Zeit auf denselben Punkt gemäß dem Bestrahlungsplan bezogen sind. Anderenfalls besteht die Gefahr, daß der Patient durch einen unkontrollierten Bestrahlungsvorgang Schäden erleidet.

Ziel der Erfindung ist es daher, eine Vorrichtung und ein Verfahren zum Steuern einer Bestrahlungseinrichtung zu schaffen, welche eine exakte, schnelle und zuverlässige Steuerung des Strahles gewährleisten. Dieses Ziel wird durch eine Vorrichtung und ein Verfahren gemäß den Ansprüchen erreicht.

Erfindungsgemäß weist die Vorrichtung zum Steuern einer Bestrahlungseinrichtung eine Kette von Schaltungsmodulen auf, wobei jedes Schaltungsmodul eine separate Kommunikationsverbindung zu einem Vorgänger-Schaltungsmodul und eine separate Kommunikationsverbindung zu einem Nachfolger-Schaltungsmodul aufweist.

Bei dem erfindungsgemäßen Verfahren zum Steuern einer Bestrahlungseinrichtung mittels von in einer Kette angeordneten Steuerungsmodulen kommuniziert jedes Steuerungsmodul separat mit einem Vorgänger-Steuerungsmodul und separat mit einem Nachfolger-Steuerungsmodul.

Die erfindungsgemäße Vorrichtung und das erfindungsgemäße Verfahren sind mit einer Reihe von Vorteilen verbunden. Durch das Vorsehen einer separaten Kornmunikationsverbindung zu einem Vorgänger-Schaltungsmodul und einer separaten Kommunikationsverbindung zu einem Nachfolger-Schaltungsmodul wird ein zuverlässiger Datenaustausch zwischen jeweils zwei Schaltungsmodulen ermöglicht. Darüber hinaus ist die Kommunikation der übrigen Schaltungsmodulpaare hiervon autonom. Dies hat eine Separation des Datenwegs und damit eine höhere Zuverlässigkeit der Vorrichtung zur Folge.

Gemäß einem bevorzugten Ausführungsbeispiel der Erfindung kann die Bestrahlungseinrichtung als eine Schwerionen-Bestrahlungseinrichtung ausgebildet sein. Damit können vorteilhafterweise besonders präzise und effektive Tumorbehandlungen durchgeführt werden.

Gemäß einem weiteren Aspekt der Erfindung kann bei der Vorrichtung mindestens eines der Schaltungsmodule eine Verbindung mit einer externen Speichereinrichtung aufweisen. Damit kann vorteilhafterweise ein Auslesen und Kontrollieren von Daten ohne Beeinflussung des Ablaufs der Strahlsteuerung durchgeführt werden.

Gemäß einem weiteren Aspekt der Erfindung können bei der Vorrichtung alle Schaltungsmodule einen gemeinsamen Triggerbus aufweisen. Vorteilhafterweise kann somit eine genaue Synchronisierung aller Schaltungsmodule erreicht werden.

Die Erfindung wird anhand der Zeichnung näher beschrieben. In der Zeichnung zeigt
Fig. 1 einen schematischen Überblick über ein Therapiebestrahlungssystem für Schwerionen gemäß einem Ausführungsbeispiel der Erfindung,
Fig. 2 einen hardwaremäßigen Überblick des Bestrahlungssystems gemäß Fig. 1,
Fig. 3 schematisch den Aufbau eines Schaltungsmoduls und
Fig. 4 die Verbindung der Schaltungsmodule miteinander und mit zugeordneten Meßeinrichtungen.

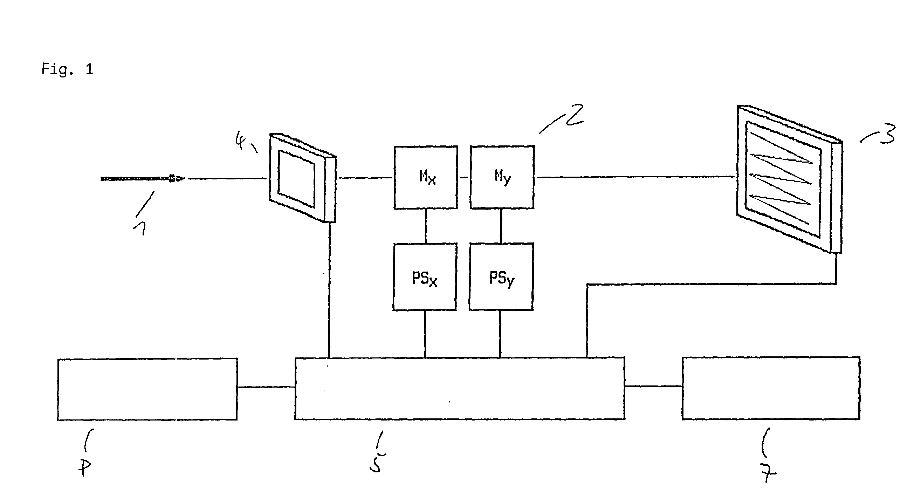

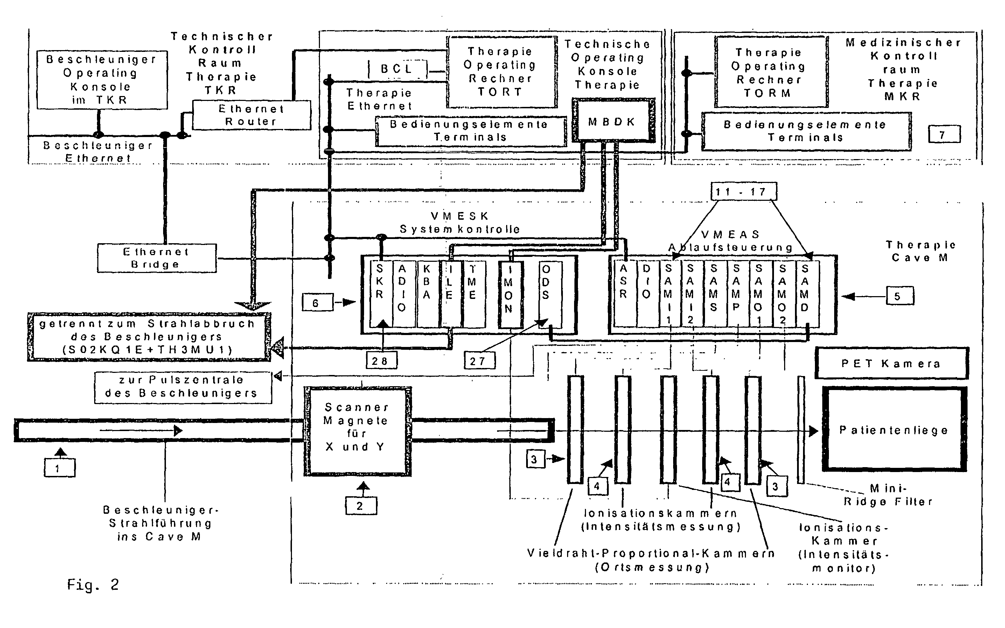

Fig. 1 gibt einen schematischen Überblick über ein Therapiebestrahlungssystem für Schwerionen gemäß einem Ausführungsbeispiel der Erfindung. Fig. 2 gibt einen hardwaremäßigen Überblick des Bestrahlungssystems gemäß Fig. 1. Der Ionenstrahl 1, erzeugt in einem nicht dargestellten Synchroton, das eine Pulszentrale P aufweist, durchtritt auf dem Weg zum Zielvolumen - dem Tumorgewebe - zunächst eine Einrichtung zur Überwachung der Strahlgeometrie, bevor er dann durch x/y-Ablenkeinheiten 2 auf einen bestimmten Punkt gelenkt wird. Bevor der Strahl in das Gewebe eintritt, durchtritt er eine Einrichtung 3 zur Überwachung der Strahlposition und Strahlbreite sowie eine Einrichtung 4 zur Überwachung der Strahlintensität. Als Ablenkeinheiten werden Elektromagnete verwendet. Die Einrichtung 4 kann auch vor den X/Y-Ablenkeinheiten angeordnet sein.

Steuerung und Überwachung des Bestrahlungssystems werden durch ein komplexes elektronisches System gewährleistet.

Das Steuerungs- und Überwachungssystem setzt sich aus drei Ebenen zusammen, nämlich einer Ablaufsteuerung 5, einer Systemkontrolle 6 und einer Bedienerführung 7. Diese arbeiten unabhängig voneinander. Verteilt über alle drei Ebenen ist ein Sicherheitssystem 8 realisiert, welches eine sofortige Abschaltung des Strahls im Falle einer Störung im System gewährleistet.

Die Ablaufsteuerung 5 erlaubt einen Zugriff von der Bedienerführung 7 nur bei der Initialisierung, beim Start und beim Notstopp. Während der Bestrahlung agiert die Ablaufsteuerung 5 autonom. Sie erfüllt neben den Steuerungsaufgaben auch Sicherheitsfunktionen, indem die gemessenen Daten mit den Vorgaben des Bestrahlungsplans verglichen werden und bei Abweichungen oberhalb festgelegter Grenzen zur Abschaltung des Strahls führen.

Die Systemkontrolle 6 erlaubt die Einstellung der Betriebsparameter, z.B. der Detektorspannungen. Des weiteren überwacht die Systemkontrolle 6 "langsam" ablaufende Vorgänge durch Auslesen einer Vielzahl von Systemzuständen und schaltet gegebenenfalls den Strahl ab.

Die Bedienerführung 7 ermöglicht dem Bediener die Interaktion mit dem Steuerungs- und Überwachungssystem. Von hier aus werden Bestrahlungspläne in die Ablaufsteuerung 5 geladen, Bestrahlungen gestartet, gestoppt oder unterbrochen, Bedieneraktionen und Systemparameter protokolliert, der Bestrahlungsvorgang und der Systemzustand anhand der Meßdaten visualisiert und die gemessenen Daten zu Dokumentationszwecken archiviert.

Das Steuerungs- und Überwachungssystem ist als VME-Umgebung realisiert und weist Bedienungseinrichtungen wie Ein-/Ausgabegeräte (Terminals) sowie eine Rechenanlage, bestehend aus mehreren Einzelrechern, mit den üblichen Peripheriegeräten auf. Die Einrichtungen zur Überwachung des Strahls bezüglich Ort, Breite und Intensität sowie die Einrichtungen zur Anforderung und Ablenkung des Strahls sind über Busverbindungen an die VME-Umgebung gekoppelt.

Das von der Ablaufsteuerung 5 unabhängig arbeitende Sicherheitssystem überwacht den Bestrahlungsvorgang während der gesamten Dauer der Bestrahlung. Es unterbricht den Bestrahlungsvorgang selbsttätig, wenn infolge einer Fehlfunktion die Ablenkung des Strahles fehlerhaft ist oder die Teilchenzahl für einen Punkt, für eine Schicht oder die insgesamt aufgebrachte Teilchenzahl überschritten wird. Hierbei kann die Ursache einer Fehlfunktion bereits in der Strahlerzeugung liegen oder in der Ablaufsteuerung 5 begründet sein, wobei die Ablaufsteuerung 5 jedoch selbst Kontrollmittel zur Unterbrechung des Bestrahlungsvorganges aufweist.

Die Ablaufsteuerung 5 weist Schaltungsmodule (Steuerungs- und Auslesemodule) 11-17 auf, die durch einen gemeinsamen Systembus 100 mit den Bedienungseinrichtungen verbunden sind. Der Systembus 100 ist als VME-Bus ausgebildet.

Jedes der Steuerungsmodule 11-17 ist jeweils durch einen separaten Gerätebus 211-217 mit einer Meßeinrichtung (Ionisationskammer, Vieldrahtkammer) 111-116 bzw. mit einer externen Speichervorrichtung 27 verbunden. Die Gerätebusse 211-217 sind von dem Systembus 100 unabhängig.

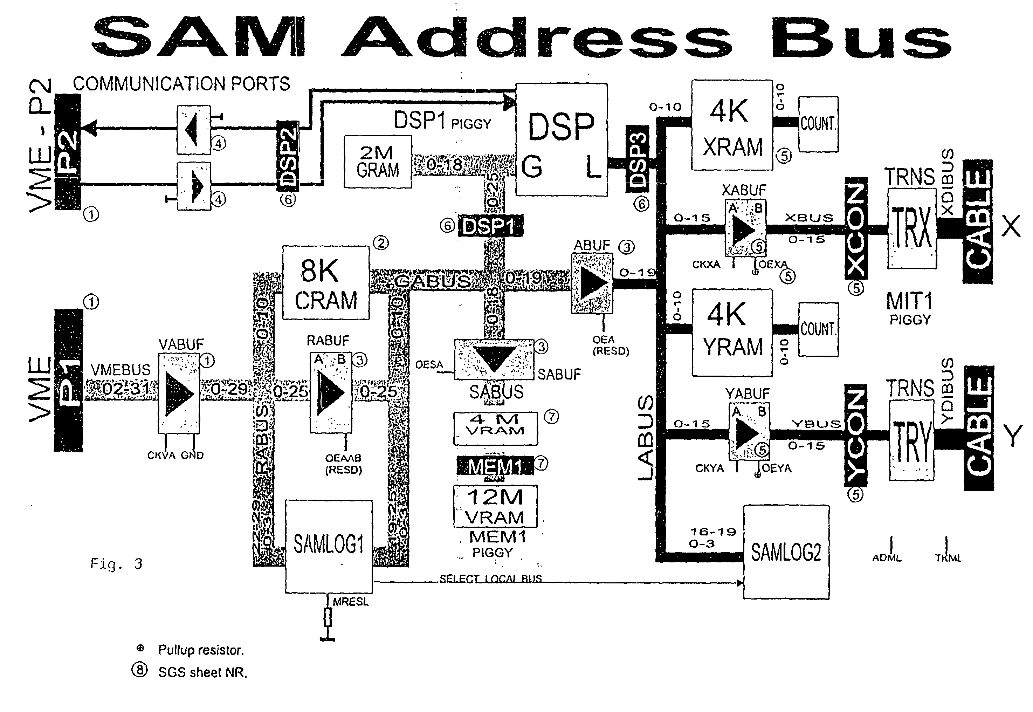

Fig. 3 zeigt schematisch den Aufbau eines Schaltungsmoduls 11-17. Jedes Schaltungsmodul enthält einen Mikroprozessor DSP, mindestens einen Lese/Schreib-Speicher MEM 1 und mindestens eine Eingabe/Ausgabe-Schnittstelle P1, P2. Vorteilhafterweise kann somit jedes Schaltungsmodul autonom arbeiten und benötigt keine zentrale Steuerung.

Mittels der Schaltungsmodule 11-17 wird die Bestrahlungseinrichtung gesteuert.

Zur Erzielung einer erhöhten Systemzuverlässigkeit sind die Orts- und die Teilchenzahlsmeßeinrichtungen mit den zugeordneten Schaltungsmodulen in der Ablaufsteuerung 5 redundant vorhanden. Hierdurch können Betriebsstörungen durch zufällige Fehler in einer Meßeinrichtung weitestgehend vermieden werden.

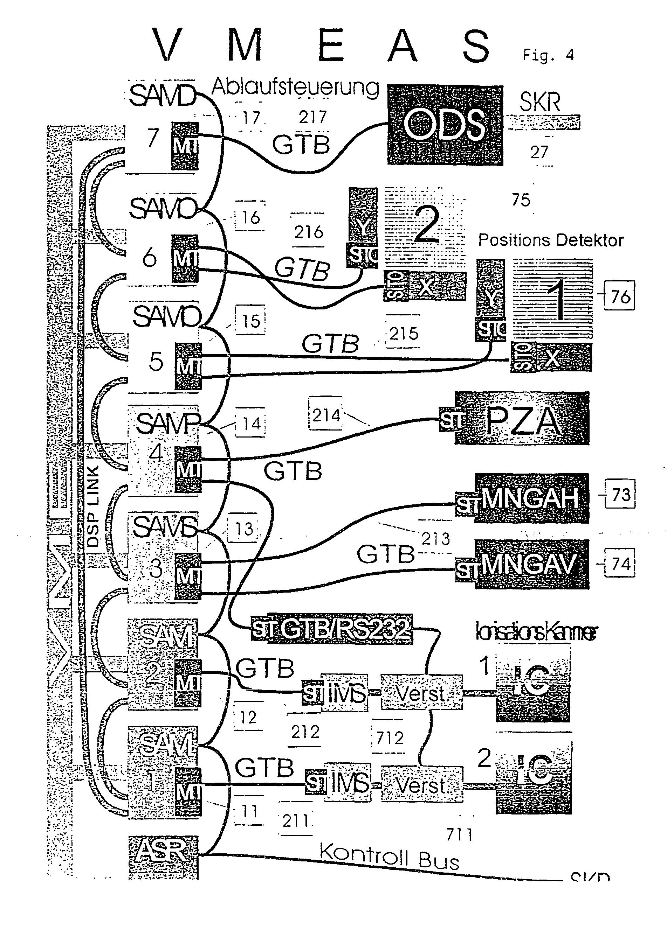

Fig. 4 zeigt die Verbindungen der Schaltungsmodule miteinander und mit den zugeordneten Meßeinrichtungen. Zur Ablenkung des Strahls auf einen Punkt sind Ablenkmagnete 2 vorgesehen - siehe Fig. 2 -, die auf den Strahl wirken. Die Ablenkung erfolgt in einer Ebenen senkrecht zur Ausbreitungsrichtung (horizontal/ vertikal). Die Ablenkmagnete werden von Magnetnetzgeräten 73, 74 mit elektrischer Energie versorgt. Angesteuert werden die Ablenkmagnete durch das Schaltungsmodul 13 der Ablaufsteuerung 5. Das Schaltungsmodul 13 und die Ablenkmagnete 2 sind durch einen Gerätebus miteinander verbunden. Die Ablenkung des Strahls ist durch Stromwerte zur Ansteuerung der beiden Ablenkmagnete 2 eindeutig definiert.

Zur Messung der tatsächlichen Ablenkung des Strahles sind zwei Positions-Detektoreinrichtungen 3 vorgesehen, die im Strahlengang angeordnet sind. Die Positions-Detektoreinrichtungen weisen jeweils eine gasgefüllte Vieldraht-Kammer auf, welche den Durchtrittsort des Strahles und die Strahlbreite erfaßt, indem an kontaktierten Gittern Ladungen gesammelt und elektronisch weiterverarbeitet werden. Der Durchtrittsort des Strahles wird mittels der an den Kammern angeordneten Elektronik 75, 76 in ein elektrisches Signal umgewandelt, welches über einen Gerätebus das Schaltungsmodul 15 bzw. 16 übermittelt wird. Der tatsächliche Strahlort wird in regelmäßigen_Zeitabständen ermittelt und mit dem Sollwert gemäß dem Bestrahlungsplan verglichen. Diese Zeitabstände sind beliebig einstellbar; ein typischer Wert hierfür ist 150 µsec.

Vorteilhafterweise können somit der tatsächliche Strahlort und die tatsächliche Strahlbreite ständig überwacht werden. Es besteht dann die Möglichkeit, den Strahl bei einer Inkonsistenz mit einem Sollwert sofort abzuschalten.

Zur Bestimmung der durch den Strahl gelieferten Teilchenzahl sind zwei Ionisationskammern 4 vorgesehen, die im Strahlengang angeordnet sind. Die beiden Ionisationskammern gleichen einander. Jede Ionisationskammer liefert ein Stromsignal, dessen Amplitude ein Maß für die gerade durchtretenden Ionen ist, an einen nachgeschalteten Strom/Spannungsverstärker 711, 712. Das dort austretende Spannungssignal wird in Zeitabständen von 12,8 µsec abgetastet und in einen Digitalwert umgewandelt. Die Digitalwerte werden zur Bestimmung der Strahlintensität mittels des Mikroprozessors - eines digitalen Signalprozessors - im Schaltungsmodul 11 bzw. 12 aufaddiert.

Vorteilhafterweise kann somit die Teilchenzahl in kurzen Zeitabständen gemessen werden und auf eine etwaige für den Patienten schädliche Überdosis sofort reagiert werden.

Die Ablaufsteuerung 5 arbeitet auf der Basis eines Datensatzes, der alle für den Bestrahlungsvorgang gemäß dem Bestrahlungsplan notwendigen Parameter enthält. Hierzu gehören insbesondere die zu bestrahlenden Punkte und die diesen Punkten zugeordneten Teilchenzahlen sowie Grenzwerte. Die Punkte sind hierbei durch die Stromwerte zur Ansteuerung der Ablenkungsmagnete des Strahlführungssystems definiert, während sich die zugeordneten Teilchenzahlen durch die Zeitdauern der Ablenkung des Strahls auf den jeweiligen Punkt ergeben.

Weiterhin mit Bezug auf Fig. 4 wird nunmehr die Kommunikationsweise der einzelnen Schaltungsmodule 11-17 beschrieben. In der Initialisierungsphase wird der Datensatz für die Steuerung und Überwachung der therapeutischen Bestrahlung eines Patienten an jedes der Schaltungsmodule 11-17 durch den Systembus 100 gesendet und dort im Speicher eines jeden der Schaltungsmodule 11-17 abgelegt. Somit verfügt jedes Schaltungsmodul 11-17 über den gleichen Datensatz. Nach der Initialisierungsphase wird die Systembus-Verbindung zu den Schaltungsmodulen 11-17 unterbrochen, d.h. die Schaltungsmodule 11-17 arbeiten während des Bestrahlungsvorganges autonom und unbeeinflußt von der übrigen Ablaufsteuerung. Dies hat den Vorteil, daß die Möglichkeit einer Störungseinwirkung von der übrigen Ablaufsteuerung auf die Schaltungsmodule 11-17 ausgeschlossen ist.

Der Bestrahlungsvorgang wird durch sequentielle Abarbeitung des Bestrahlungsplans Punkt für Punkt durch die Schaltungsmodule 11-17 der Ablaufsteuerung 5 gemäß der Rasterscan-Methode gesteuert.

Für die Abarbeitung eines Punktes i liest das erste Schaltungsmodul 11, welches die Teilchenzahl überwacht, aus dem in seinem Speicher abgelegten Datensatz den oder die Parameter (z.B. Soll-Teilchenzahl) und mißt die Teilchenzahl des auf diesen Punkt i gelenkten Strahles. Sobald die Soll-Teilchenzahl für den Punkt i erreicht ist, wird von Schaltungsmodul 11 ein Triggersignal auf den Triggerbus gesetzt, wodurch die Schaltungsmodule 11-16 veranlaßt werden, die von ihnen jeweils benötigten Parameter für den nächsten Punkt i+1 aus ihren Speichern zu lesen. Gleichzeitig sendet das Schaltungsmodul 11 alle Parameter für diesen nächsten Punkt i+1 über eine Prozessor-Schnittstelle an das Nachfolger-Schaltungsmodul 12, wobei es die gemessene Teilchenzahl an die Stelle des betreffenden Parameters setzt. Das Nachfolger-Schaltungsmodul 12 vergleicht den bzw. die aus seinem Speicher gelesenen Parameter, die es zur Abarbeitung des Punktes i+1 benötigt (z.B. wiederum eine Soll-Teilchenzahl), mit dem bzw. den vom Vorgänger-Schaltungsmodul empfangenen Parametern und legt alle empfangenen Parameter in seinen Speicher an die Stelle der Parameter für den betreffenden Punkt i+1. Stimmen die verglichenen Parameter überein, so kann davon ausgegangen werden, daß der Speicherinhalt des Schaltungsmoduls 12 für den betreffenden Punkt i+1 korrekt war und das Schaltungsmodul 12 mit korrekten Parametern arbeitet. Stimmen die beiden verglichenen Parameter nicht überein, so gibt das Nachfolger-Schaltungsmodul 12 seine Parameter für den nächsten Punkt zwar weiter, jedoch wird eine Fehlermeldung ausgegeben, wodurch die die Bestrahlung sofort unterbrochen wird.

Dieser Ablauf setzt sich sinngemäß für alle Schaltungsmodule 12 bis zum Schaltungsmodul 17 am oberen Ende der Kette fort.

Am oberen Ende der Schaltungsmodulkette ist ein Auslese-Schaltungsmodul 17 angeordnet, welches den Empfang der Parameter von seinem Vorgänger-Schaltungsmodul 16 erwartet.

Diese Parameter enthalten nunmehr nicht mehr die Parameter-Sollwerte, sondern die von den einzelnen Schaltungsmodulen gemessenen Werte für den Punkt i. Dieser Datensatz wird von Schaltungsmodul 17 über einen Gerätebus an eine externe Speichervorrichtung 27 zur Speicherung weitergeleitet.

Gleichzeitig wird dieser Datensatz über eine separate Busverbindung an das erste Schaltungsmodul 11 zurückgesendet.

Nunmehr setzt sich der Ablauf für den Punkt i+1 in der Weise fort, wie sie oben für den Punkt i beschrieben ist.

Trifft der Datensatz jedoch nicht innerhalb derjenigen Zeit ein, die für die Teilchenzahl gemäß Bestrahlungsplan notwendig ist, so ist anzunehmen, daß mindestens einer der gesendeten Parameter des Punktes i+1 innerhalb der Kette der Schaltungsmodule 11-17 inkonsistent ist. Trifft nämlich der Datensatz am Schaltungsmodul 11 erst ein, wenn es bereits die Gesamtteilchenzahl für den Punkt i registriert hat und somit bereits der Punkt i+1 bestrahlt wird, geht die Synchronisierung verloren. In diesem Fall gibt das Schaltungsmodul 11 eine Fehlermeldung aus, wodurch der Bestrahlungsvorgang unterbrochen wird.

In der Ablaufsteuerung 5 werden vorteilhafterweise also mehrere Funktionen gleichzeitig erfüllt:
- Alle Schaltungsmodule enthalten den gleichen Datensatz, d.h., die Schaltungsmodule arbeiten konsistent.
- Alle Schaltungsmodule arbeiten jeweils denselben Punkt ab, d.h., sie arbeiten synchron miteinander.
- Durch den Vergleich der Daten wird überprüft, ob in den einzelnen Schaltungsmodulen Speicherfehler vorliegen.
- Durch das Überschreiben eines Parameters mit einem Meßwert beim Senden der Parameter wird das Sammeln und Ausgeben von Meßwerten während des Bestrahlungsvorganges ermöglicht.

Die externe Speichervorrichtung 27 ist über den Systembus mit dem Steuer- und Kontrollrechner 28 mit den anderen Komponenten der Systemsteuerung verbunden. Am Steuer- und Kontrollrechner 28 kann der Bestrahlungsverlauf visualisiert werden. Dies räumt dem Bediener, etwa dem behandelnden Arzt, die Möglichkeit ein, den Bestrahlungsverlauf zu verfolgen bzw. manuell zu unterbrechen.

Das unabhängig von der Ablaufsteuerung 5 arbeitende Sicherheitssystem garantiert eine zusätzliche Überwachung des Bestrahlungsvorganges. Wesentlich hierbei ist eine zusätzliche Überwachung der Teilchenzahl für jeden Punkt. Hierzu ist eine dritte Ionisationskammer vorgesehen, die in Anordnung und Funktion den beiden anderen Ionisationskammern 4 gleicht. Mittels dieser dritten Ionisationskammer wird neben der momentanen Teilchenzahl (für einen Punkt) auch der Gesamtbetrag für jeweils eine Ebene und alle Ebenen ermittelt. Hierzu wird für jeden Bestrahlungspunkt die Ladung aus der Ionisationskammer 4 gesammelt, in eine Impulsfolge gewandelt und in einem Zähler aufaddiert. Der momentane Zählerstand wird ständig mit dem höchstzulässigen Wert für die betreffende Ebene verglichen. Ist der Sollwert überschritten, so wird eine Fehlermeldung ausgegeben und der Bestrahlungsvorgang abgebrochen. In ähnlicher Weise wird in einem weiteren Zähler die gesamte Teilchenzahl, d.h. die Summe der Teilchenzahlen über alle Ebenen gebildet. Auch hier erfolgt ständig ein Vergleich mit dem höchstzulässigen Gesamtwert, um eine Überdosis zu vermeiden.

## Patentansprüche

1. Vorrichtung zum Steuern einer Bestrahlungseinrichtung, aufweisend:
eine Kette von Schaltungsmodulen (11-17), wobei jedes Schaltungsmodul (11-17) eine separate Kommunikationsverbindung zu einem Vorgänger-Schaltungsmodul und
eine separate Kommunikationsverbindung zu einem Nachfolger-Schaltungsmodul aufweist.

2. Vorrichtung nach Anspruch 1, **dadurch gekennzeichnet, daß** die Bestrahlungseinrichtung eine Schwerionen Bestrahlungseinrichtung ist.

3. Vorrichtung nach Anspruch 1 oder 2, **dadurch gekennzeichnet, daß** die Kommunikationsverbindungen jeweils als Busse ausgebildet sind.

4. Vorrichtung nach einem der vorhergehenden Ansprüche, **dadurch gekennzeichnet, daß** jedes der Schaltungsmodule (11-17) einen Mikroprozessor, einen Lose/Schreib-Speicher und mindestens eine Eingabe/Ausgabe-Schnittstelle aufweist.

5. Vorrichtung nach einem der vorhergehenden Ansprüche, **dadurch gekennzeichnet, daß** jedes Schaltungsmodul (11-17) einen Gerätebus zur Verbindung mit jeweils mindestens einem externen Gerät aufweist.

6. Vorrichtung nach einem der vorhergehenden Ansprüche, **dadurch gekennzeichnet, daß** mindestens eines der Schaltungsmodule (11-17) eine Verbindung (217) mit einer externen Speichereinrichtung (27) aufweist.

7. Vorrichtung nach einem der vorhergehenden Ansprüche, **dadurch gekennzeichnet, daß** alle Schaltungsmodule (11-17) einen gemeinsamen Triggerbus aufweisen.

8. Vorrichtung nach einem der vorhergehenden Ansprüche, **dadurch gekennzeichnet, daß** die Vorrichtung zur Steuerung und Überwachung von Intensität und Ablenkung eines Strahles ausgebildet ist.

9. Bestrahlungseinrichtung mit einer Vorrichtung nach einem der vorhergehenden Ansprüche.

10. Verfahren zum Steuern einer Bestrahlungseinrichtung mittels von in einer Kette angeordneten Steuerungsmodulen (11-17), wobei jedes Steuerungsmodul (11-17) separat mit einem Vorgänger-Steuerungsmodul und separat mit einem Nachfolger-Steuerungsmodul kommuniziert.

11. Verfahren nach Anspruch 10, **dadurch gekennzeichnet, daß** die Bestrahlungseinrichtung eine Schwerionen-Bestrahlungseinrichtung ist.

12. Verfahren nach Anspruch 10 oder 11, **dadurch gekennzeichnet, daß** die Steuerungsmodule (11-17) über Busse kommunizieren.

13. Verfahren nach einem der Ansprüche 10 bis 12, **dadurch gekennzeichnet, daß** jedes Schaltungsmodul (11-17) durch einen Gerätebus jeweils mindestens ein externes Gerät steuert.

14. Verfahren nach einem der Ansprüche 10 bis 13, **dadurch gekennzeichnet, daß** mindestens eines der Schaltungsmodule Daten an eine separate Speichereinrichtung (27) sendet.

15. Verfahren nach einem der Ansprüche 10 bis 14, **dadurch gekennzeichnet, daß** alle Steuerungsmodule (11-17) gemeinsam getriggert werden.

16. Verfahren nach einem der Ansprüche 10 bis 15, **dadurch gekennzeichnet, daß** mindestens ein Steuerungsmodul (11; 12) die Intensität und mindestens ein Steuerungsmodul (13, 15, 16) die Ablenkung des Strahles für einen zu bestrahlenden Punkt steuert und überwacht.

17. Verfahren nach einem der Ansprüche 10 bis 16, **dadurch gekennzeichnet, daß** jedes Steuerungsmodul (11-17) einen Satz von Steuerparametern für die Bestrahlungseinrichtung speichert.

18. Verfahren nach einem der Ansprüche 10 bis 17, **dadurch gekennzeichnet, daß** jedes Steuerungsmodul (11-17) jeweils mindestens einen Parameter des Satzes von Parametern für einen Punkt durch einen Meßwert ersetzt und den Satz an das Nachfolger- Steuerungsmodul überträgt und daß das Nachfolger- Steuerungsmodul mindestens einen Parameter des übertragenen Satzes eines Punktes mit jeweils dem entsprechenden mindestens einen Parameter des gespeicherten Satzes desselben Punktes vergleicht.

## Claims

1. An apparatus for controlling a radiation device, comprising: a chain of circuit modules (11-17), each circuit module (11-17) having a separate communication connection to a preceding circuit module and a separate communication connection to a following circuit module.

2. An apparatus according to claim 1, **characterised in that** the radiation device is a heavy ion radiation device.

3. An apparatus according to claim 1 or 2, **characterised in that** in each case the communication connections are in the form of buses.

4. An apparatus according to one of the preceding claims, **characterised in that** each circuit module (11-17) comprises a micro processor, a read/write memory, and at least one input/output interface.

5. An apparatus according to one of the preceding claims, **characterised in that** each circuit module (11-17) comprises an apparatus bus for connection with, in each case, at least one external apparatus.

6. An apparatus according to one of the preceding claims, **characterised in that** at least one of the circuit modules (11-17) has a connection (217) to an external memory facility (27).

7. An apparatus according to one of the preceding claims, **characterised in that** all circuit modules (11-17) have a common trigger bus.

8. An apparatus according to one of the preceding claims, **characterised in that** the apparatus is designed for controlling and monitoring the intensity and deflection of a beam.

9. A radiation device having an apparatus according to one of the preceding claims.

10. A method for controlling a radiation device by means of control modules (11-17) disposed in a chain, wherein each control module (11-17) communicates separately with a preceding control module and separately with a following control module.

11. A method according to claim 10, **characterised in that** the radiation device is a heavy ion radiation device.

12. A method according to claim 10 or 11, **characterised in that** the control modules (11-17) communicate via buses.

13. A method according to one of claims 10 to 12, **characterised in that** each circuit module (11-17) controls at least one respective external apparatus by means of an apparatus bus.

14. A method according to one of claims 10 to 13, **characterised in that** at least one of the circuit modules transmits data to a separate memory facility (27).

15. A method according to one of claims 10 to 14, **characterised in that** all control modules (11-17) are triggered commonly.

16. A method according to one of claims 10 to 15, **characterised in that** at least one control module (11; 12) controls and monitors the intensity and at least one control module (13, 15, 16) controls and monitors the deflection of the beam for a point to be irradiated.

17. A method according to one of claims 10 to 16, **characterised in that** each control module (11-17) stores a set of control parameters for the radiation device.

18. A method according to one of claims 10 to 17, **characterised in that** each control module (11-17) replaces at least one parameter of the set of parameters for a point with a measured value and transmits the set to the following control module, and the following control module compares at least one parameter of the transmitted set of a point with in each case the corresponding at least one parameter of the stored set of the same point.

## Revendications

1. Dispositif pour commander une installation d'irradiation, comportant :
une chaîne de modules de commutation (11-17) sachant que chaque module de commutation (11-17) comporte une liaison de communication séparée à un module de commutation précédent et une liaison de communication séparée à un module de commutation suivant.

2. Dispositif selon la revendication 1, **caractérisé en ce que** l'installation d'irradiation est une installation d'irradiation par ions lourds.

3. Dispositif selon la revendication 1 ou 2, **caractérisé en ce que** les liaisons de communication sont configurées respectivement comme des bus.

4. Dispositif selon l'une quelconque des revendications précédentes, **caractérisé en ce que** chacun des modules de commutation (11-17) comporte un microprocesseur, une mémoire de lecture et d'écriture et au moins une interface d'entrée/sortie.

5. Dispositif selon l'une quelconque des revendications précédentes, **caractérisé en ce que** chaque module de commutation (11-17) comporte un bus d'appareil pour la liaison avec au moins un appareil externe respectivement.

6. Dispositif selon l'une quelconque des revendications précédentes, **caractérisé en ce qu'**au moins un des modules de commutation (11-17) comporte une liaison (217) avec un dispositif à mémoire externe (27).

7. Dispositif selon l'une quelconque des revendications précédentes, **caractérisé en ce que** tous les modules de commutation (11-17) présentent un bus de déclenchement commun.

8. Dispositif selon l'une quelconque des revendications précédentes, **caractérisé en ce que** le dispositif est configuré pour la commande et pour la surveillance de l'intensité et de la déviation d'un faisceau.

9. Installation d'irradiation avec un dispositif selon l'une quelconque des revendications précédentes.

10. Procédé pour commander une installation d'irradiation, au moyen de modules de commutation (11-17) disposés dans une chaîne, sachant que chaque module de commutation (11-17) communique séparément avec un module de commutation précédent et séparément avec un module de commutation suivant.

11. Procédé selon la revendication 10, **caractérisé en ce que** l'installation d'irradiation est une installation d'irradiation par ions lourds.

12. Procédé selon la revendication 10 ou 11, **caractérisé en ce que** les modules de commutation (11-17) communiquent par des bus.

13. Procédé selon l'une quelconque des revendications 10 à 12, **caractérisé en ce que** chaque module de commutation (11-17) commande au moins un appareil externe par un bus d'appareil.

14. Procédé selon l'une quelconque des revendications 10 à 13, **caractérisé en ce qu'**au moins un des modules de commande émet des données à un dispositif à mémoire séparé.

15. Procédé selon l'une quelconque des revendications 10 à 14, **caractérisé en ce que** tous les modules de commande (11-17) sont déclenchés en commun.

16. Procédé selon l'une quelconque des revendications 10 à 15, **caractérisé en ce qu'**au moins un module de commande (11 ; 12) commande et surveille l'intensité et qu'au moins un module de commande (13, 15, 16) commande et surveille la déviation du faisceau pour un point à irradier.

17. Procédé selon l'une quelconque des revendications 10 à 16, **caractérisé en ce que** chaque module de commande (11-17) enregistre un jeu de paramètres de commande pour l'installation d'irradiation.

18. Procédé selon l'une quelconque des revendications 10 à 17, **caractérisé en ce que** chaque module de commande (11-17) remplace respectivement au moins un paramètre du jeu de paramètres pour un point par une valeur de mesure et transmet le jeu au module de commande suivant et **en ce que** le module de commande suivant compare au moins un paramètre du jeu transmis d'un point au respectivement au moins un paramètre correspondant du jeu enregistré du même point.
